# EUROPEAN PATENT SPECIFICATION

(11) **EP 1 478 246 B1**
(45) Date of publication and mention of the grant of the patent: **10.10.2007**
(21) Application number: 03708126.2
(22) Date of filing: 24.02.2003
(51) Int. Cl.: A23L 1/30, A61K 35/66, A61K 35/74, A23L 1/03, C12N 1/20, C12N 1/16

(54) **DIETETIC AND/OR PHARMACEUTICAL COMPOSITIONS FOR HUMAN AND/OR ANIMAL USE BASED ON PROBIOTIC MICROBIAL PREPARATIONS**
VON MENSCHEN ODER TIEREN VERWENBARE DIÄTETISCHE UND/ODER PHARMAZEUTISCHE ZUBEREITUNGEN, AUF BASIS VON PROBIOTISCHEN MIKROORGANISMEN
COMPOSITIONS DIETETIQUES ET/OU PHARMACEUTIQUES DESTINEES A L'HUMAIN ET/OU L'ANIMAL, A BASE DE PREPARATIONS MICROBIENNES PROBIOTIQUES

(30) Priority: 28.02.2002 IT MI20020399
(43) Date of publication of application: 24.11.2004
(73) Proprietor: Centro Sperimentale del Latte S.P.A., 20131 Milan (IT)
(72) Inventor: BIANCHI SALVADORI, Bruna, I-20121 Milan (IT); PIROVANO, Franco, I-20050 Sulbiate (IT); ROTTIGNI, Claudio, I-20121 Milan (IT)
(74) Representative: Martegani, Franco
(86) International application number: PCT/EP2003/001867
(87) International publication number: WO 2003/071883

(56) References cited:
- EP-A- 1 177 794
- WO-A-93/06208
- WO-A-99/62348
- WO-A-02/060276
- RU-C- 2 178 975
- US-A- 5 716 615
- GIROLA M ET AL: "Efficacy of probiotic preparation with living, freeze-dried lactic acid bacteria and yeast on child diarrhoea" BIOSIS, XP002035135
- GARDINER G ET AL: "EVALUATION OF CHEDDAR CHEESE AS A FOOD CARRIER FOR DELIVERY OF A PROBIOTIC STRAIN TO THE GASTROINTESTINAL TRACT" JOURNAL OF DAIRY SCIENCE, AMERICAN DAIRY SCIENCE ASSOCIATION. CHAMPAIGN, ILLINOIS, US, vol. 82, no. 7, July 1999 (1999-07), pages 1379-1387, XP000850203 ISSN: 0022-0302
- FAMULARO G ET AL: "TRADITIONAL AND HIGH POTENCY PROBIOTIC PREPARATIONS FOR ORAL BACTERIOTHERAPY" BIODRUGS, AUCKLAND, NZ, vol. 12, no. 6, 1999, pages 455-470, XP009001219 ISSN: 1173-8804

## Description

The present invention relates to dietetic and/or pharmaceutical compositions for human and/or animal use based on probiotic microbial preparations.

In particular, the present invention relates to dietetic and/or pharmaceutical compositions for human and/or animal use and foodstuffs in general, based on microbial cultures consisting of autochthonous and allochthonous species with respect to human beings and animals, selected from the lactic bacterial species *Lactobacillus acidophilus, Lactobacillus plantarum, Lactobacillus casei* subsp. *casei, Lactobacillus casei* subsp. *rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii* subsp. *delbrueckii, Lactobacillus* delbrueckii and all its subspecies, Lactobacillus *gasseri, Lactobacillus johnsonii, Streptococcus thermophilus, Lactobacillus delbrueckii* subsp. *bulgaricus*, optionally associated with *Streptococcus thermophilus; Lactobacillus fermentum, Lactobacillus brevis, Lactococcus lactis* subsp. *lactis, Lactococcus lactis subsp. cremoris and Leuconostoc spp.; Entercoccus faecium, Pediococcus pentosaceus, Pediococcus acidilactici; Bifidobacteria such as Bifidobacterium Longum, Bi - fidobacterium Breve, Bifidobacterium bifidum, Bifidobacterium infantis, Bifidobacterium lactis;* and/or propioni-bacterial species, yeast species and/or mold species; the above species being live and vital and/or devitalized, and said species being present in microbial cultures in a dried concentrated form with a concentration ranging from 10⁶ ufc/g to 10¹¹ ufc/g, said compositions being also characterized in that they comprise different strains of the same species with a different sensitivity to bacteriophages (lysogeny and lysotypy) and with the same biological and probiotic properties.

The above-mentioned probiotic micro-organisms shall be indicated hereafter with the term "pr micro-organisms".

The compositions, object of the present invention, have an equilibrating action of the intestinal flora of the host (human being and animal), in addition to producing various beneficial/probiotic effects towards the organism which vary according to the destination target, such as children, adults, elderly people, expectant women, persons with various kinds of deficiencies or gas-tro-intestinal disturbances (dismicrobisms) or with acute or chronic diseases, for example of the vaginal or urological type.

These components, with a reciprocal association, act according to a biological and synergic succession on an intestinal level.

It is also known that bacteriophages and bacteria form a more or less indissoluble pair to the extent that it can be declared that there are no micro-organisms without bacteria. The development of bacteriophages is generally considered as being a production problem, but it can also be observed in intestinal flora after the ingestion of microbial preparations which exert a probiotic effect. In this case, the problem must be solved in the product formulation phase so as to preserve the positive effects of the composition.

It has been found that the most effective solution consists in the differentiation of the species and availability, for each of these, of numerous strains with a different sensitivity to bacteriophages so that all the species are always present in the intestines.

If, on the contrary, the product only consists of a single strain, it is not possible to guarantee the presence of all the species in the case of lysis of this strain and the beneficial effects obtainable with their ingestion will be lost.

The positive effects of a bacterial culture consisting of a single strain of a certain species are consequently rather limited as it can be easily attacked by its specific bacteriophages.

Another factor which can inhibit the development of bacteriophages is the administration of preparations for oral bacterium therapy for quite a limited period of time in order to reduce the possibility of the development of bacteriophages.

When preparations for oral bacterium-therapy are administered for very prolonged periods of time to a large number of individuals, as is the case for example in hospitals, the risk of phagic infection is extremely high. The problem can be solved by substituting the strains used with other strains definitely resistant to those specific bacteriophages.

Bacteriophages are an extremely important biological reality, even more widely diffused than micro-organisms themselves. Cases of diarrhea not due to the action of pathogenous germs can be correlated to the phagic attack of some normal constituents of intestinal flora. It should be pointed out that phages are harmless for human beings even though they may interfere with the intestinal flora.

The continuous administration of bacteria of the same strain, with a probiotic action, leads to the development of specific bacteriophages which destroy said bacterial strain, thus annulling its probiotic prophylaxis. A knowledge of bacteriophages can be of help in the preparation and use of cultures adopted in oral bacterium-therapy.

WO02/060276 discloses the use of two strains of *Lactobacillus rhamnosus* LGG and LC705. It is specified that the first is *Lactobacillus rhamnosus* GG (LGG) and the second is *Lactobacillus casei subsp. Rhamnosus* LC705, therefore these are strains of different species, characterised by a different use of sugars.
In fact, *Lactobacillus rhamnosus* GG (LGG) does not ferment lactose and it exhibits excellent adhesion to both mucus and epithelial cells, while *Lactobacillus casei subsp. Rhamnosus* LC705 adheres weakly to mucus cells, but moderately to epithelial cells.
WO93/06208 discloses the use of two strains *Enterococcus faecium* 301 and *Enterococcus faecium* 202 and indicates that it is not known precisely why these two organisms provide the desirable features of the invention, namely increased rate of weight gain, better feed conversion, increased yield of breast meat, and increased uniformity of flock weight. The fact is that they do, provided that both are used in combination so that they can somehow interact with each other, and providing that they are used within the range herein expressed. The two strains *Enterococcus faecium* 301 and *Enterococcus faecium* 202 must act in symbiosis to be able to carry out their improved function. US-A-5,716,615 discloses a pharmaceutical composition containing several different bacteria including *Streptococcus Thermophilus, Lactobacilli* and *Bifidobacteria.*
In the abstract of "Efficacy of probiotic preparation with living, freeze-dried lactic acid bacteria and yeast on child diarrhoea" BIOSIS, Girola M. et al., it is disclosed a clinical study about the effect on child diarrhoea of a preparation with living, freeze-dried lactic acid bacteria and yeasts.
RU-A-2178975 discloses a product containing as bacterial mass the symbiotic system of, at least, two strains of lactic-acid bacteria named *Lactobacillus acidophilus.* It, also, contains antioxidants as biologically active substances, namely: fermentative complex obtained from a biomass of cultivated cells or superoxide dismutase. Additionally, it contains either a vitamin or vitamins which possess an antioxidant activity: vitamins A, C, E. It improves functions of gastrointestinal tract and state of skin surfaces.
The, at least, two strains of lactic-acid bacteria named *Lactobacillus acidophilus* are a symbiotic system and they do not obviously share the same biological and probiotic properties. In fact, they must act in symbiosis to be able to carry out their improved function.

In order to obtain a effective protection with respect to intestinal disturbances, the problem of bacteriophages has been considered and solved with the composition according to the present invention.

The present invention relates to a composition which comprises different strains of the same species having a different sensitivity to bacteriophages (lysogeny and lysotypy) but with the same biological and probiotic properties.

The composition according to the present invention can also comprise at least one of the following components: other micro-organisms, enzymes, mineral salts, vitamins, prebiotics, natural fibres, phyto-derivatives, antioxidants, fermented milk, paps, feeds.

The live and vital and/or devitalized yeasts of the compositions, object of the present invention, are yeasts with a low fermentative capacity for probiotic use, rich in essential amino acids. In particular, the yeast can be *Saccharomyces cerevisiae* or *Saccharomyces boulardii.*

In the composition according to the present invention, at least one of the pr micro-organisms is preferably present in a concentration lower than 10⁹ ufc/g.

In particular, a dietetic and/or pharmaceutical composition according to the present invention comprises *Streptococcus thermophilus,* Bifidobacteria such as *Bifidobacterium longum, Bifidobacterium breve, Bifidobacterium infantis, Bifidobacterium lactis, Bifidobacterium bifidum, Lactobacillus acidophilus* in a concentration ranging from 10⁹ to 10¹¹ ufc/g, *Lactobacillus plantarum, Lactobacillus casei* subsp. *casei, Lactobacillus delbrueckii* subsp. *bulgaricus, Enterococcus faecium* in a concentration ranging from 10⁶ to 10⁹ ufc/g.

In particular, *S*. *thermophilus* and *L. delbrueckii* subsp. *bulgaricus* are developed in symbiosis (or proto-cooperation).

The natural enzymes used essentially consist of a mixture made up of β-glucanase and xylanase produced by micro-organisms of the Trichoderma type.

The mixture comprising these natural enzymes is particularly useful for optimizing the digestive catalase of hydrosoluble polysaccharides (NSP). This mixture develops a synergic action on the various NSP contained in wheat, barley, oats, rye and triticum, and is defined by its specific efficacy on the various substrates, the analytical characteristics of the single components, the product properties studied for commercial use in association with a pool of pr micro-organisms in human and animal nutrition.

The natural fibres, possibly present in the composition according to the present invention, are selected from fibres of acacia, oats, apples, inulin, psyllium, microcrystalline cellulose (which act as prebiotics).

The composition according to the present invention comprising pr micro-organisms and prebiotics, such as natural fibres, is of particular interest.

The fibres represent a nutritional substrate for the pr micro-organisms. The metabolic activity of pr micro-organisms on the fibres (prebiotic) makes them easier to digest and causes the free release of substances useful for the nutrition of the organism (human or animal), of the pr micro-organisms and autochthonous intestinal flora.

The phyto-derivatives are preferably selected from extracts from Eleuterococcus and green tea.

The antioxidants are preferably natural antioxidants and can be selected from oleuropein (from extravirgin olive oil), lycopene (from tomatoes), bioflavonoids (from citrus fruit), phenol components (from red grapes).

The vitamins and mineral salts are selected from vitamin A, B1, B2, B6, B12, niacin, C, D3, E, folic acid, calcium, phosphorous, magnesium, iron, zinc.

A further object of the present invention therefore relates to the use of said dietetic and/or pharmaceutical compositions as integrators and/or dietetic-therapeutic products for human and/or animal nutrition.

In particular, an object of the present invention also relates to the use of said dietetic and/or pharmaceutical compositions for preparing integrators, dietetic-therapeutic food products, food, drinks and/or feeds for human and/or animal nutrition.

The food can consist of milk, cheese, paps, homogenized products (based on meat, milk, cheese, fruit, vegetables), dietetic food products destined for diabetics such as jams, chocolate, sweeteners other than sucrose, or animal feeds.

The milk can be fermented or non-fermented milk, with the direct inoculum of suitable pr micro-organisms in a concentrated dried form.

The pr micro-organisms in a concentrated dried form inoculated into milk are suitable for the processing of 1000 or more liters of milk without any intermediate passage with the presence on the finished product of the probiotics identified.

Therapeutic dietetic cheese can be obtained by the addition of suitable pr micro-organisms in a concentrated dried form in a certain processing phase of the cheese in order to guarantee, in a certain gram-weight of cheese, the supply of the dose of pr micro-organisms necessary for the organism.

The drinks can be instantaneous drinks or water containing the compositions according to the present invention.

The present patent application also relates to integrators, food, dietetic-therapeutic food products, drinks and/or feeds for human and/or animal nutrition, characterized in that they contain a dietetic and/or pharmaceutical composition according to the present invention.

The use of the compositions according to the present invention for human nutrition has an equilibrating action on the intestinal flora of the host (human being and animal), in addition to producing various beneficial/probiotic effects towards the organism which depends on the destination target (children, adults, elderly people, expectant women, people with various kinds of deficiencies or gastro-intestinal disturbances i.e. dismicrobisms, or with acute or chronic diseases, for example of the vaginal or urological type). This use also regulates intestinal functioning, improves constipation, hemorrhoids and intestinal irritation; it reduces the absorption of sugar and cholesterol.

The use of the compositions according to the present invention in zootechnics allows growth promotion, the control of enteric pathologies of a bacterial origin, an improvement in the digestive efficiency (I.C.A.) of animals for breeding, the food conversion index and an improvement in the quality of the end-product (meat or eggs, in the case of fowl), thus solving problems linked to antibiotic residues.

In addition to this, the use of the compositions according to the present invention results in a better consistency of feces, a reduction in enteric pathologies of a bacterial origin (colibacillosis), an improvement in the consistency of the egg-shells produced, an improvement in the egg-laying itself, a reduction in therapeutic treatment and an improved immunitary response.

In particular, as far as human nutrition is concerned, the compositions according to the present invention, containing suitable live and vital and/or devitalized pr micro-organisms in concentrated dried form, can be used as a dietetic and/or therapeutic product in powder form as tablets, capsules or sachets both for pharmaceutical use and as food integrators, optionally combined with natural principles.

Pr micro-organisms can, in fact, interact with nutritional principles of a natural origin such as fibres of acacia, oats, apples, Psyllium - which already have health-giving properties and whose reduced food contribution can be correlated with various pathologies, such as constipation, obeseness, cardiovascular diseases, diabetes.

These substances, also classified as prebiotics, interact with pr micro-organisms (such as lactic bacteria and bifidobacteria) as they act as a substrate on which the pr micro-organisms themselves develop thus creating a synergic effect. The symbiotic combination between pr micro-organisms and prebiotic fibres, such as inulin, favours intestinal functioning, improves constipation, hemorrhoids and intestinal irritation; it reduces the absorption of sugar and cholesterol.

Another extremely interesting combination is that between pr micro-organisms and antioxidants of a natural origin, such as: bioflavonoids, anthocyanins, lycopene, orthophenols, extracts from citrus fruit, red grapes, tomatoes, olive oil, respectively. These antioxidants are not only effective in fighting AOR (Active Oxygen Radicals), but they are also capable of protecting the integrity and functionality of microflora on an intestinal level, guaranteeing a greater beneficial effect on human beings.

Pr micro-organisms can also be "coupled" with vitamins and minerals, especially in cases where a lack of these is likely. Deficiencies in specific trace elements are now associated with chronic health problems: typical examples are fluorine and dental decay, chromium and tolerance to glucose, copper and hypercholesterolemia, zinc and the immunitary system.

The association of vitamins and minerals with pr micro-organisms improves the absorption, i.e. bioavailability, of nutritional principles, a factor which is often neglected in evaluating the composition of integrators and/or the necessity for their use. A second important aspect is linked to the action synergy, for example between zinc and pr micro-organisms which are both implied in the modulation of the immunitary function.

This synergy also develops between various species of vegetable substances, phyto-derivatives (extracts from Eleuterococcus and green tea), known for their tonic, adaptogenous, antistress, immuno-potentializing activity, but at the same time a hypocholesterolemizing, antioxidant activity, for controlling glycemia, modulating the ecosanoid cascade, with safe health effects.

The combination with pr micro-organisms favours the effect of phyto-estrogens as they increase the bioavailability and absorption of these vegetable principles, in addition to normalizing the equilibrium of intestinal flora altered in situations of stress.

Pr micro-organisms in association can also be used for enriching dietetic food products destined for children and adults, for example in homogenized products (based on meat, milk, cheese, fruit, vegetables, etc.) or in products destined for diabetics (jams, chocolate, sweeteners other than sucrose), etc.

The pr micro-organisms can be mixed with the above food preparations, with fermented milk and with food products in general, in different proportions.

The addition to food of pr micro-organisms in concentrated dried form is justified in that these products have equilibrating properties of intestinal flora and stimulate the immunological properties and natural defence system of the organism in addition to those against tumours.

In animal nutrition, on the other hand, the compositions according to the present invention, containing live and vital and/or devitalized pr micro-organisms, in a dried concentrated form, can be used for the following purposes.

The biological processes linked to microbial life present with respect to the enteron are extremely important for animals, as they can influence both the digestive processes and also the absorption processes of the nutritional principles. In this respect, two main types of micro-organisms can be distinguished on an intestinal level: fermentation micro-organisms which produce, starting from glucosides, various short-chain fatty acids and putrefaction micro-organisms which degrade the amino acids producing biogenic amines. Under normal physiological conditions, the putrefaction processes are controlled by fermentative processes, whereas when there is an enteric pathology, bacterial strains with a high putrefactive action, above all *Escherichia coli,* are predominant.

In this particular context, resort is made to the use of selected cultures of pr micro-organisms which form the typical and most effective antagonist of putrefactive flora. The antagonist action is in fact carried out by means of a "barrier effect", exerted with adhesion to the intestinal epithelium, and an acidifying activity which makes the enteric environment unsuitable for the development of pathogenous flora. Suitable specifically combined pr micro-organisms allow the development of a beneficial microflora to be activated on an intestinal level. This is an innovative technology which is extremely effective and completely without side-effects, for controlling enteritis of a bacterial origin and for improving the digestion of breeding animals, the food conversion index and problems linked to residues in meat or eggs (in the case of fowl). In particular, the following selection criteria are adopted for selecting the strains which form these cultures:
- adhesion capacity to the epithelium of the enteron;
- resistance to gastric acidity;
- development and production rate of lactic acid at the level of the enteron;
- processing of the enzymes useful for food degradation.

100,000,000 quintals of feed form the basic datum for evaluating the potential market of selected cultures of pr micro-organisms used as growth promoters and as active principles for the prophylaxis of enteric diseases. In both cases, the advantages in terms of quality improvement (in particular of meat, reduction in the use of antibiotics and consequently the elimination of residues) which products based on pr micro-organisms offer, are important.

In addition to a greater control of enteric pathologies of a bacterial origin and an improvement in the digestive efficiency (I.C.A.), the application of lactic bacteria shows:
- an improved consistency of feces;
- a reduction in enteric pathologies of a bacterial origin (colibacillosis);
- an improvement in the consistency of the egg-shells produced;
- an improvement in the laying;
- a reduction in therapeutic treatment;
- an improvement in the immunitary response.

In particular, technology development in animal nutrition is increasingly emphasizing the problem of the use of antibiotics for auxinic purposes in feeds.

The pros and cons, advantages and disadvantages, use and abuse of these molecules have often been the object of discussion between scientists, which frequently involves the public. In England, the "Swam Institute" published a report in 1969 in which the use of antibiotics for zootechnical productions was connected to the risk of allergic or toxic reactions, and to the danger of the formation of antibiotic-resistant microbial strains, with cases of a wide diffusion of resistance to said antibiotics from animals to human beings. When antibiotics are absorbed by animals, in fact, they can be found in the form of residues in meat.

There is also the problem relating to possible intolerance on the part of those working in feed producers and of zootechnical operators, who can come into contact with the various active principles.

The identification and use of natural auxinic factors which do not produce negative effects such as those mentioned above, is therefore of great interest.

The use of gastrointestinal flora "modulators", such as yeasts, lactic bacteria and bifidobacteria, is becoming more and more well known in the preparation of food destined for animal nutrition of the concentrated type or in the form of fodder.

Limitations in the use of these products derive from the current legislations in force which only allow the use of some species of pr micro-organisms (lactic bacteria and yeasts), at very low concentrations, which often jeopardize the efficacy in zootechnical applications.

Detailed studies in the selection and preparation of new species of pr micro-organisms suitable for use in various zootechnical species, are consequently extremely important.

Another differentiation to be taken into consideration as a selection criterion of these products is the different action mechanism according to the animal species, distinguishing between monogastric species and those with a polygastric digestive system.

The formulations of the composition according to the present invention are polyvalent and therefore play an important role in the preventive treatment of gastric and intestinal diseases.

These preparations contain not only pathogen agent antagonists, but also compounds which produce biologically active substances for regulating the metabolic processes in animals and raising their resistance to infections.

Said compositions consist of a mixture of pr micro-organisms (for example, lactic bacteria, propionibacteria and yeasts) in a concentrated and dried form.

Lactic bacteria are in fact antagonists of many pathogenous varieties and are vitamin producers, raising the resistance of the organism to illnesses. Propionibacteria (which form part of the intestinal microflora of ruminants) produce propionic acid (important for the regular growth of calves), acetic acid (essential for the synthesis of milk fat, during lactation) and B12 vitamins.

For yeasts, biomasses have been selected and produced, which due to their biochemical characteristics, increase their probiotic properties and are functional for zootechnical use, also identifying the specificity for the different animal species and various productions. This solves the present situation whereby yeasts destined for zootechnical use consist, in the best of cases, of surpluses of yeast production for bread-making, with the selection and production of yeasts which therefore have:
- a high adaptability under the conditions present in the digestive system of animals;
- a high content of particular cellular elements (amino acids, vitamins, etc.).
Yeasts favour a series of important biochemical reactions in the rumen, which improve the fermentative activity, allowing the contemporaneous presence, in milk cows, of the three volatile fatty acids precursors of milk components (fat, casein and lactose), in the highest possible concentrations.

In the zootechnical field, in particular as far as cattle breeding is concerned, it has been demonstrated that autochthonous microflora affects the animal's health as it can participate in the digestion and metabolism of the nutritive substances, supplying energy, amino acids and sugar which could otherwise not be available to the host.

An appropriate equilibrium between the autochthonous micro-organisms normally present in the intestines or rumen, ensures, among other things, the mutual beneficial relationship between host and microflora and consequently the health of the animal.

As a result, the treatment of animals with the polyvalent compositions according to the present invention, consisting of a mixture of pr micro-organisms (for example lactic bacteria, propionibacteria and yeasts), creates not only an antagonistic action against pathogenous agents, but also the production of biologically active substances which regulate the metabolic processes of animals and raise their resistance to infections.

To actuate similar treatment in intensive breeding it is necessary however to have cultures which are highly concentrated in cells and are dried, to facilitate transportation and use.

In conclusion, the use of the compositions according to the present invention containing pr micro-organisms in a concentrated and dried form to be used as food integrators for cattle leads to:
- an improvement in the state of health of animals with a consequent increase in meat yield;
- a reduction in the use of antibiotics in feeding breeding cattle, with indirect effects for consumption due to a greater hygienic reliability of the meat;
- positive ecological consequences on the waste water disposal of farms together with the advantages already mentioned and deriving from the concentrated and dried form (multiple and simple use and easy transportation).

According to research effected on fowl species (hens, broilers, turkeys) it has been found that the administration of specific cultures of pr micro-organisms, is extremely effective from the very first day of the animal's life.

The development of a totally beneficial intestinal flora and consequently the start of the productive cycle under optimal functioning conditions of the digestive system, has, in fact, been observed.

The use of pr micro-organisms in animal breeding is extremely practical as it can be effected both via the food (feed) and also via the drinking water, care being taken not to subject the pr micro-organisms to temperatures higher than 65-70°C, so as not to jeopardize their vitality.

The pr micro-organisms selected for the compositions object of the present patent application have different nutritional demands and require the application of a wide variety of fermentative parameters (growth temperatures, pH conditions, fermentation times, drying curves, etc.) for which a general production scheme is provided below (Scheme 1)

SCHEME 1 – Process diagram

Pure strains from
strain library
(Microbiology laboratory)

Raw materials
(Warehouse)

Vegetative phase
Preparation of pre-inoculum
(Microbiology laboratory)

Weighing of medium ingredients
(Preparation department)

Preparation, dissolution and pasteurization
or sterilization of the cultural medium
(Production dept.)

Primary fermentation
Incubation of pre-inoculum
200/400 liters
(Production dept.)

Final fermentation
Incubation of inoculum
5,000/10,000 liters
Production dept.)

Concentration by centrifugation or ultra-filtration or
micro-filtration, etc..
(Production dept.)

Homogenization with cryo-protective products
(Production dept.)

Raw materials
(Warehouse)

Mixing
(Production dept.)

Drying
(Spraying, freeze drying, zeolite, etc..)
(Production dept.)

Microbial culture in dried concentrated form

For illustrative purposes only, the manufacturing process is described below of one of the components of the mixtures of pr micro-organisms such as the species *Streptococcus thermophilus.*

The manufacturing process comprises a vegetative phase in a flask, starting from collection strains (in a laboratory), a production phase including primary and final fermentation and the actual production phase (concentration, homogenization, drying).

### Vegetative phase

### Preparation of the pre-inoculum

Different strains of the same species can be used for the manufacturing of the species *S*. *thermophilus*.

For the preparation of the pre-inoculum (vegetative phase) the contents of the various phials containing the collection strains (1 per strain) are dissolved separately with 1.5 ml of a solution of peptone and sterile salt. The suspensions contained in the phials are mixed and inoculated in equal quantities into 10 ml test-tubes of sterile skimmed milk or dried milk re-formed at 10% in water. They are incubated at 37°C until the milk coagulates or until the logarithmic growth phase of the culture thus obtained (first culture).

The first culture is transferred to a 250 ml flask of sterile skimmed milk. It is incubated at 42°C until coagulation (second culture) and, if necessary, it can be conserved at 5°C for 3 days.

The contents of the second culture are transferred (in a ratio of 2% = 20 ml/l) into a flask containing 4 l (or 8 l in relation to the expected fermentation volume) of a substrate of re-formed sterile skimmed milk in a ratio of 10% and containing 1% of yeast extract and incubated at 37°C for about 2 hours (until coagulation). The flask is then left to cool in a refrigerator (4°C) until use. The phase contrast microscopic morphological control, the growth and acidification curve control and the control of the contaminating germs and acidity developed are then contemporaneously effected on the third culture (laboratory preinoculum).

### 1^{st} production phase

### Primary fermentation

This phase is carried out in a 200/400 litre fermentor using 200 l (or 400 l in relation to the expected fermentation volume) of dried skimmed milk re-formed at 8% (containing 0.1% of Antifoam additive), as culture medium. The components of the medium were previously weighed and dissolved, using an automatic plant. Pasteurization of the culture medium is then effected, under slow stirring, at 90°C for 30 minutes. The medium is cooled to 42-44°C and is sterilely inoculated with the contents of the flask (preinoculum, 4 or 81) of the vegetative phase. The fermentation is continued for 2-3 hours, after which rapid cooling is initiated to 4-8°C with icy water.

The culture is maintained at 5°C until the end of the quality control.

### Final fermentation

### Preparation of the culture medium

Culture medium 5000 l (or 10000 l).

A 5000 l fermentor is used (in the case of 10000 l the quantities are doubled), previously washed and flushed with a CIP cycle. 4400 liters of water are charged, and the fermentor is heated to 60°C.
The following products are weighed:
- Lactose 80 Kg
- Yeast extract 25 Kg
- Dextrose 150 Kg
- Ammonium sulfate 15 Kg
- Monobasic potassium phosphate 7.5 Kg
- Manganese sulfate 0.5 Kg
- Antifoam additive 0.5 Kg

The components of the medium are dissolved using an automatic plant. Pasteurization is then effected, under slow stirring, at 80°C for 30 minutes.

### Inoculation and incubation

The culture medium mass is cooled to 40-42°C; it is sterilely inoculated with 200 liters of the primary fermentation culture and is thermostat-regulated; it is kept under stirring and incubated for 3-4 hours approximately, controlling the pH which is continuously adjusted with NaOH at 30% to maintain a value of 6.1-6.2. The regulation is effected continuously, under stirring.

Various samplings are taken for the different controls and rapid cooling to 5°C with icy water, is initiated. The operations are carried out so as to complete the fermentation within the same day, enabling the microbiological purity control (e.g. absence of coliforms) to be effected before the operations of the following day.

### 2^{nd} production phase

### Concentration of the cellular mass (e.g. by centrifugation)

### Preparation of the centrifuges

The Westphalia centrifuges (or ALFA LAVAL) are washed and flushed using a CIP plant with NaOH and nitric acid, washed with hot water and pre-cooled (hollow cavity) with icy water to 5°C.

The culture to be concentrated is directly transferred, by means of a lobe pump, to the centrifuges, using a closed circuit plant, flushed in CIP, maintaining a flow-rate of 1000 1/hour (2500 1/hour for ALFA LAVAL). The first three discharges of concentrate are eliminated and the whole of the remaining quantity is directly transferred, still in line, to the homogenizer.

### Homogenization of the concentrate with cryoprotective products

The concentrate collected is added with a cryoprotective product (in a ratio of 16% with respect to the weight of the concentrate collected) and homogenized. The cryoprotective product is thus composed: (dose per 300 Kg of concentrate):
- Dried skimmed milk 4.0 Kg
- F.U. Lactose 12.8 Kg
- Sucrose 4.0 Kg
- Yeast extract 3.2 Kg
- Water 24.0 l
(the cryoprotective product is previously prepared and pasteurized at 80°C), the pH is corrected to 6.3-6.5 with NaOH at 30%, samplings are taken for the controls and it is transferred to a vacuum freeze drier.

At the end of the operations, the centrifuge is immediately flushed with nitric acid - soda in CIP, and is rinsed, first with rinsing water and then with hot water. The homogenizer is immediately flushed with nitric acid - soda and water at 90°C.

### Drying (e.g. by vacuum freeze drying)

All the vacuum freeze drying accessories must be flushed, before use, in compliance with the specific procedures whereas the chambers and fixed parts are decontaminated with vapour and/or a solution of sodium hypochlorite or another suitable decontaminant.

The concentrate is directly distributed by means of a pump to the vacuum freeze drying basins, stratifying about 1.5 cm per basin (also in relation to the actual quantity of material obtained). The vacuum freeze drying process is activated according to the automatic program of the lyostat; the vacuum freeze drying is considered as being terminated (normally after about 36-40 hours) when all the temperature registration curves of the product are stable for at least 8 hours and the chamber isolation test does not reveal vacuum drops higher than 10% (less than 100 microbar).

The basins are extracted from the lyostat, the product is immediately discharged and the lyophilized product is collected in double polyethylene bags. The product is ginned on an oscillating blade granulator previously washed and equipped with an inox grid of 2.77 x 1 mm and collected in double polyethylene bags or in steel drums. Each drum or bag is sampled for control and immediately placed in a quarantine cell. At the end of the drying process, the yield % to dried product obtained is calculated, with respect to the damp concentrate.

### Final mixing (standardization and stabilization)

The product destined for the use listed above can consist of the mixture of several pr micro-organisms in a predefined cellular ratio. This predefinition obviously cannot be actuated in a hypothetical fermentation in a mixed culture but can only be effected from an appropriate mixing of the single species which takes into account the single cellular numerical charges of the single components.

Through said mixing, (in addition to the necessary "titre" standardization according to specifications) the result of a stabilization of the cellular numerical charge is reached, together with its ratios between the various species. For this purpose, suitable inert diluents are used (such as F.U. Lactose or, alternatively and for use in lactose-free specialties, maize starch, SiO₂, Mg Stearate).

The mixing is effected using a 750/1000 l biconic mixer, previously washed and flushed with a suitable decontaminating agent, by means of a validated mixing cycle. The diluent can be added in two or three successive aliquots, in relation to the relative quantities.

At the end of the mixing, the product is discharged into double polyethylene bags, and immediately sampled for specific quality controls; the bags are immediately thermo-sealed and placed, in adequate packaging, in the quarantine area of the refrigerator at +2/+8°C "Awaiting Analysis".

Some typical schemes are provided below for the preparation of mixtures standardized and stabilized with a predetermined titre. As they are raw materials to be dosed at a *minimum vital cellular charge,* (UFC - Unit Forming Colonies), the quantities naturally vary slightly from lot to lot, whereas the diluent must, necessarily, be calculated each time according to the a.n. (as necessary) scheme.

### Scheme a) (total microbial charge not less than 300 billion cells/g)

### 200 Kg Lot

| *Species* | *(Minimum) UFC Quantity* |
|---|---|
| *S. thermophilus* | 4.6x10¹⁶ |
| Bifidobacteria | 2.1x10¹⁶ |
| *L. acidophilus* | 4.4x10¹⁴ |
| *L. delbrueckii* subsp. *bulgaricus* | 0.68x10¹⁴ |
| *L. plantarum* | 0.50x10¹⁴ |
| *L. casei* | 0.50x10¹⁴ |
| *E. faecium* | 0.068x10¹⁴ (minimum 50 g) |
| F.U. Lactose a.n. | 200 Kg |

### Scheme b) (total microbial charge not less than 100 billion cells/g)

### 200 Kg Lot

| *Species* | *(Minimum) UFC Quantity* |
|---|---|
| *S. thermophil us* | 1.54x10¹⁶ |
| Bifidobacteria | 0.7x10¹⁶ |
| *L. acidophilus* | 1.47x10¹⁴ |
| *L. delbrueckii* subsp. *bulgaricus* | 0.234x10¹⁴ |
| *L. plantarum* | 0,174x10¹⁴ |
| *L. casei* | 0.174x10¹⁴ |
| *E. faecium* | 0.023x10¹⁴ (minimum 50 g) |
| F.U. Lactose a.n. | 200 Kg |

### Scheme c) (total microbial charge not less than 70 billion cells/g - Lactose free)

### 200 Kg Lot

| *Species* | *(Minimum) UFC Quantity* |
|---|---|
| *S. thermophilus* | 1.07x10¹⁶ |
| Bifidobacteria | 4.9x10¹⁵ |
| *L. acidophilus* | 1.03x10¹⁴ |
| *L. delbrueckii* subsp. *bulgaricus* | 0.16x10¹⁴ |
| *L. plantarum* | 0.11x10¹⁴ |
| *L. casei* | 0.11x10¹⁴ |
| *E. faecium* | 0.016x10¹⁴ (minimum 50 g) |
| SiO₂ | 2.0 Kg |
| Mg Stearate | 1.0 Kg |
| F.U. Lactose a.n. | 200 Kg |

The quality control of the formulation after mixing is effected as described below.

Control of the differential microbial charge in the mixture.

Peptone-salt diluent: 1 g of peptone and 8.5 g of NaCl are dissolved in 1000 ml of water; the mixture is stirred carefully, heated and, if necessary, the pH is adjusted so as to obtain, after sterilization in an autoclave, a value of 7.0 at 25°C.

Method: 10 g approximately, carefully weighed, of dried concentrated culture are diluted to 100 ml (dilution 1:10) with the diluent solution.

The mixture is stirred carefully and homogenized in a Stomacher homogenizer for 1.5 minutes and the cells are left to revitalize in a thermostat at 37°C for 20 minutes. 10 ml of the first dilution (10⁻¹) are transferred to an empty sterile bottle, diluted with 90 ml of diluent (10⁻²) and stirred carefully. The decimal dilutions are repeated in succession to 10⁻⁹; the dilutions thus obtained will be used in triplicate form.

Preparation of the plates for the counting of the pr micro-organisms: Three replicates per dilution to be examined are distributed on an adequate number of 90 mm Petri plates, together with 14 ml of HHD commercial broth, containing 20 g/l of agar and 1 g/l of Tween 80. The mixture is left to cool and dried under a horizontal flow hood. 0.1 ml of the pre-selected dilution is planted at the centre of each plate and is distributed by palet-ting/rotation with a 70-75 mm glass spatula until the complete absorption of the planting. The plates are incubated in anaerobiosis with a Gas-Pack system for 72 hours at 37°C

The counting is effected (referring to the different dilutions) by distinguishing the various species on the basis of the different morphologies, colouring of the colony and by means of stereoscopic microscopic control. When there are doubts, a part of the colony in question is removed with a needle and a microscopic preparation is transferred to a sample slide with a drop of sterile water and is covered with a covering slide. It is observed in phase contrast with a 40x lens and a 10x eyepiece (for Bifidobacteria a 100x immersion lens is used).

### Examples of compositions based on probiotic microbial preparations for human and/or animal use.

The examples are provided for illustrative purposes only and in no way limit the scope of the present invention.

### EXAMPLE 1

Homogenized products destined for children 1 g of live and vital *L*. *bulgaricus* and *S*. *thermophilus* (in concentrations of 1 to 5x10⁹ ufc/g), in symbiotic association, in a dried concentrated form are mixed with 50-100 grams of homogenized product.

### EXAMPLE 2

Food integrators based on live and vital lactic bacteria and bifidobacteria.

These integrators contain the following live and vital pr micro-organisms:

| | | |
|---|---|---|
| *St. thermophilus* | ~ 27% equal to | 540x10⁶ ufc/g |
| Bifidobacteria | ~ 20% | 400x10⁶ ufc/g |
| *Lc. lactis, Lc. cremoris, Leuconostoc* sp | | ~5.5 % |
| | equal to | 110x10⁶ ufc/g |
| *L. casei* | ~ 8% | 100x10⁶ ufc/g |
| *L. helveticus* | ~ 5.5% | 110x10⁶ ufc/g |
| *L. plantarum* | ~ 37% | 740x10⁶ ufc/g |
| | Total 100% Total | 2000x10⁶ ufc/g |

and also contain, according to the use and for illustrative purposes, the following compounds:
A) natural fibres (fibres of acacia, oats, apples, inulin, psyllium, microcrystalline cellulose) for regulating the intestines and weight control.

The integrator thus obtained is particularly suitable for hypocaloric diets, constipation, hemorrhoidal disturbances, for the prevention of obesity, varicose veins, intestinal irritation;

### B) natural antioxidants for free radical defence, and anti-aging effect.

The antioxidants comprise oleuropein (from extravirgin olive oil), lycopene (from tomatoes), bioflavonoids (from citrus fruits), phenol components (from red grapes). The integrator thus obtained is suitable for people subject to oxidative stress deriving from unbalanced nutrition, a disorderly life-style, cigarette smoke and pollution, it is also suitable for the prevention of degenerative and cardiovascular diseases and against deterioration of the cellular functions, including aging;
C) vitamins and mineral salts for the re-equilibrium of the intestinal flora with a supply of nutritional substances.

The nutritional substances comprise vitamins A, B1, B2, B6, B12, niacin, C, D3, E, folic acid, calcium, phosphorous, magnesium, iron, zinc.

This combination is suitable for people following hypocaloric diets and do-it-yourself dieters, those who carry out moderate or intense sporting activities, elderly people and inappetent children, adolescents in the development phase, expectant women and during breast-feeding, smokers, after the administration of antibiotics and as a preventive treatment for osteoporosis;
D) vitamin C, Siberian ginseng (Eleuterococcus), ginger and green tea for increasing resistance to stress, and controlling intestinal problems correlated therewith. The integrator thus obtained is suitable for people who travel widely, live in situations of any kind of stress (family, work, studies and life-stile), managers and for improving physical efficiency and protection against infective diseases.

### EXAMPLE 3

Fermented milk prepared with a mixture of lactic bacteria and bifidobacteria.

A 20-30 g sachet of dried concentrated product is thus composed:

| | |
|---|---|
| *S. thermophilus + L. delbrueckii* substp. *bulgaricus* grown in symbiosis | 50x10⁹ ufc/g |
| *L. acidophilus* | 10x10⁹ ufc/g |
| *L. casei* | 50x10⁹ ufc/g |
| *L. plantarum* | 10x10⁹ ufc/g |
| Bifidobacteria | 50x10⁹ ufc/g |

This composition is inoculated directly into 1000 liters of milk previously pasteurized and cooled to a temperature of 44°C. It is left to incubate at this temperature for the time necessary for the development of the micro-organisms inoculated (7-8 h) and at the end of the process, a fermented milk is obtained with high probiotic qualities, having a pH of 4.1-4.3 and containing per ml:

| | |
|---|---|
| 1000-2000 x 10⁶ ufc | *S. thermophilus* |
| 100-200 x 10⁶ ufc | *L. delbrueckii* subsp. *bulgaricus* |
| 10-100 x 10⁶ ufc | *L. acidophilus* |
| 10-100 x 10⁶ ufc | *L. casei* |
| 10-100 x 10⁶ ufc | *L. plantarum* |
| 50-100 x 10⁶ ufc | bifidobacteria. |

### EXAMPLE 4

Food integrators for broilers based on lactic bacteria.

These integrators contain the following lactic bacteria:

| | |
|---|---|
| *L. acidophilus* | 10x10⁹ ufc/g |
| *L. plantarum* | 50x10⁹ ufc/g |
| *S. thermophilus*/*L. bulgaricus* | 2.5x10⁹ ufc/g |
| *L. salivarius* | 1x10⁹ ufc/g |

Excipient: lactose

100 g of product are administered to drinking water and this quantity of product is sufficient for 10,000 subjects.

### EXAMPLE 5

Feed for broilers based on lactic bacteria as in Example 4 with the addition of exogenous enzymes. 1 g of exogenous enzymes/kg of feed is used.

This mixture allows the hydrolysis of specific glucosides both of the non-starchy type and also those partially indigestible. It develops a synergic effect with the enzymes in the catabolism of polysaccharides of wheat, barley, oats, rye and triticum and contemporaneously integrates the intestinal flora of the animals.

The administration to broilers of both preparations allows an improvement in the retention of nitrogen, growth and ICA; a reduction in cholesterol in the blood and cecum coliforms.

### EXAMPLE 6

Food integrator for hens based on lactic bacteria and bifidobacteria.

This integrator contains the following combination of pr micro-organisms;

| | |
|---|---|
| *L. acidophilus* | 10x10⁹ ufc/g |
| *L. plantarum* | 50x10⁹ ufc/g |
| *L. casei* | 5x10⁹ ufc/g |
| *S. thermophilus*/*L. bulgaricus* | 2.5x10⁹ ufc/g |
| *L. salivarius* | 1x10⁹ ufc/g |
| *Bifidobacterium bifidum* | 50x10⁹ ufc/g |

Excipient: lactose

100 g of product are administered to drinking water and this quantity of product is sufficient for 10,000 subjects.

### EXAMPLE 7

Feed for hens based on lactic bacteria and bifidobacteria as in Example 3 with the addition of exogenous enzymes.

1 g of exogenous enzymes/kg of feed is used.

Unlike what is specified in Example 5, this mixture contains pr micro-organisms conditioned to the catabolism of polysaccharide monomers and oligomers, in particular arabinose and xylose, particularly studied for developing a synergic effect with the exogenous enzymes in the complete catabolism of polysaccharides of wheat, barley, oats, rye and triticum.

The administration of the preparations of Example 6 and 7 to hens stimulates the animal's appetite and allows an improvement in the phytasic activity in the digestive tract, in the P retention of N and Ca, the ICA laying rate, the egg/hen mass, the specific weight of the eggs and thickness of the shells; a lowering of the pH in the goiter and intestines and cholesterol in the yoke.

### EXAMPLE 8

Preparation of an antibiotic-free integrated feed as a substitution of milk destined for calves.

| | |
|---|---|
| - lactic bacteria: | 0.5 % |
| *L. plantarum* | 2x10⁹ ufc/g |
| *E. faecium* | 2x10⁹ ufc/g |
| *S. thermophilus*/*L. bulgaricus* | 10x10⁹ ufc/g |

Excipient: lactose
- dried whey 85%
- coconut oil l8%
- fat 4%
- cereal flour 2%
- mineral and vitamin integrator 0.5%

The substitutive integrated feed has a pH equal to 5.0 after re-formation in water at 10%.

### EXAMPLE 9

Complementary feed for milk cows/meat cattle based on a mixture of pr micro-organisms.

The complementary feed comprises the following pr micro-organisms:

| | |
|---|---|
| *L. plantarum* | 10x10⁹ ufc/g |
| *S. thermophilus*/*L. bulgaricus* | 50x10⁹ ufc/g |
| Propionibacteria | 10x10⁹ ufc/g |
| *Saccharomyces cerevisiae* | 10x10⁹ ufc/g |
| *L. casei* | 10x10⁹ ufc/g |
| *L. helveticus* | 10x10⁹ ufc/g |

Excipients: cereals and their by-products to obtain a charge of 2x10⁹ ufc/g.

30-100 grams/head/day are administered. The preparation increases the ingestion of dry substances, it contributes to reducing post-birth hygiene problems, exerts a detoxifying and hepato-protective action; it increases the production of milk with a high fat and protein titre; it stimulates rumenal fermentation and favours fibre digestion.

The concentration of live yeasts, total anaerobic bacteria and celluloselytic bacteria in rumenal liquid (ufc log10/ml) gave the following results:

| | Control | Preparation |
|---|---|---|
| | | (15g/head/day) |
| - Yeasts | 5.40 | 6.87 |
| - Total anaerobic bacteria | 8.98 | 10.35 |
| - Celluloselytic bacteria | 7.28 | 8.82 |

### EXAMPLE 10

Food integrator for piglets with a mixture of lactic bacteria.

The following ingredients are introduced and mixed in a double-chamber (or V-shaped) mixer having the appropriate capacity:
- Live and vital lactic bacteria in a dried concentrated form (100x10⁹ ufc/g)

| | |
|---|---|
| *L. acidophilus* | 10x10⁹ ufc/g |
| *L. plantarum* | 50x10⁹ ufc/g |
| *S. thermophilus*/*L. bulgaricus* | 30x10⁹ ufc/g |
| *E. faecium* | 10x10⁹ ufc/g |

- A carrier consisting of lactose or whey in powder or any other form, in a quantity sufficient for obtaining a microbial charge of 2 billion cells per gram of integrator product.

The product thus obtained is introduced, in the desired quantities, into a horizontal mixer and mixed with the products forming the various feeds in the following proportions:
- dried skimmed milk 10%
- dried whey 5%
- soybean flour 10%
- maize flour 20%
- fish meal 5%
- barley flour 5%
- oat flour 2.5%
- barley flakes 20%
- oat flakes 5%
- bran 15%
- mineral salts 2%
- vitamin mixture 1%
- integrators with lactic bacteria 0.5%

## Claims

1. Dietetic and/or pharmaceutical compositions for human and/or animal use based on microbial cultures consisting of autochtonous and allochtonous species, with respect to human beings and animals, selected from the lactic acid bacteria species *Lactobacillus acidophilus, Lactobacillus plantarum*, *Lactobacillus casei* subsp. *casei, Lactobacillus casei* subsp. *rhamnosus, Lactobacillus zeae, Lactobacillus salivarius, Lactobacillus lactis, Lactobacillus helveticus*, *Lactobacillus reuteri*, *Lactobacillus amylovorus, Lactobacillus crispatus, Lactobacillus curvatus, Lactobacillus delbrueckii* subsp. *delbrueckii, Lactobacillus* delbrueckii and all its subspecies, Lactobacillus *gasseri, Lactobacillus johnsonii, Streptococcus thermophilus, Lactobacillus delbrueckii* subsp. *bulgaricus,* optionally associated with *Streptococcus thermophilus; Lactobacillus fermentum, Lactobacillus brevis, Lac*tococcus *lactis* subsp. *lactis, Lactococcus lactis* subsp. *cremoris* and *Leuconostoc* specie; *Enteococcus faecium, Pediococcus pentosaceus, Pediococcus acidilactici;* Bifidobacteria such as *Bifidobacterium longum, Bifidobacterium breve, Bifidobacterium bifidum, Bifidobacterium infantis, Bifidobacterium lactis;* and/or propionibacterium species, yeast species and/or mold species; the above species being live and viable and/or devitalized, and said species being present in microbial cultures in a dried concentrated form with a concentration ranging from 10⁶ ufc/g to 10¹¹ ufc/g,
said compositions being also **characterized in that** they comprise different strains of the same species with a different sensitivity to bacteriophages and with the same biological and probiotic properties.

2. The compositions according to claim 1, **characterized in that** they also comprise at lest one of the following components: other micro-organisms, enzymes, mineral salts, vitamins, prebiotics, natural fibres, phyto-derivatives, antioxidants, fermented milk, paps, feeds.

3. The compositions according to claim 1, **characterized in that** they comprise prebiotics, such as natural fibres.

4. The compositions according to claim 1, **characterized in that** at least one of the probiotic micro-organisms is present in a concentration lower than 10⁹ ufc/g.

5. The compositions according to claim 1, **characterized in that** they comprise *Streptococcus thermophilus,* Bifidobacteria such as *Bifidobacterium longum, Bifidobacterium breve, Bifidobacterium infantis, Bifidobacterium lactis*, *Bifidobacterium bifidum, Lactobacillus acidophilus* in a concentration ranging from 10⁹ to 10¹¹ ufc/g, *Lactobacillus plantarum*, *Lactobacillus casei* subsp. *casei*, *Lactobacillus delbrueckii* subsp. *bulgaricus, Enterococcus fae*cium in a concentration ranging from 10⁶ to 10⁹ ufc/g.

6. The compositions according to claim 1, **characterized in that** the live and viable and/or devitalized yeasts are yeasts with a low fermentative capacity for probiotic use, rich in essential amino acids.

7. The compositions according to claim 6, **characterized in that** the yeast is *Saccharomyces cerevisiae* or *Saccharomyces boulardii.*

8. The compositions according to claim 2, **characterized in that** the natural enzymes consist of a mixture made up of β-glucanase and xylanase produced by micro-organisms of the Triochoderma type.

9. The compositions according to claim 2, **characterized in that** the natural fibres are selected from fibres of acacia, oats, apples, inulin, psyllium, microcrystalline cellulose.

10. The compositions according to claim 2, **characterized in that** the antioxidants are natural antioxidants.

11. The compositions according to claim 10, **characterized in that** the natural antioxidants are selected from oleuropein (from extravirgin olive oil), lycopene (from tomatoes), bioflavonoids (from citrus fruits), phenol components (from red grapes).

12. The compositions according to claim 2, **characterized in that** the vitamins and mineral salts are selected from vitamin A, B1, B2, B6, B12, niacin, C, D3, E, folic acid, calcium, phosphorous, magnesium, iron, zinc.

13. The compositions according to claim 2, **characterized in that** the phyto-derivatives are selected from those extracted from Eleuterococcus and green tea.

14. The compositions according to claim 1, **characterized by** the presence of *S*. *thermophilus* and *L. delbrueckii* subsp. *bulgaricus* developed in symbiotic association (or proto-cooperation).

15. The use of the compositions according to any of the claims from 1 to 14, for preparing integrators, foodstuffs and/or dietetic-therapeutic products, drinks and/or feeds for human and/or animal nutrition.

16. The use according to claim 15, wherein the food products are milk, cheese, paps, homogenized products (based on meat, milk, cheese, fruit, vegetables), dietetic food products destined for diabetics such as jams, chocolate, sweeteners other than sucrose, or animal feeds.

17. The use according to claim 15, wherein the milk is a fermented or non-fermented milk, with the direct inoculum of probiotic micro-organisms in a dried concentrated form.

18. The use according to claim 15, wherein the cheese is a therapeutic dietetic cheese obtained by the addition of probiotic micro-organisms in a dried concentrate form in a certain processing phase of the cheese.

19. The use according to claim 15, wherein the drinks can be instantaneous drinks or water containing the compositions according to any of the claims from 1 to 14.

20. Integrators, foodstuffs, and/or dietetic-therapeutic products, drinks and/or feeds for human and/or animal nutrition **characterized in that** they contain a composition according to any of the claims from 1 to 14.

## Patentansprüche

1. Diätetische und/oder pharmazeutische Zusammensetzungen für humane und/oder tierische Verwendung, basierend auf mikrobiellen Kulturen, bestehend aus bezüglich Menschen und Tieren autochtonen und allochtonen Arten, ausgewählt aus den *Milchsäurebakterienarten Lactobacillus acidophilus, Lactobacillus plantarum, Lactobacillus casei* subsp. *casei, Lactobacillus casei subsp. rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii* subsp. *delbrueckii, Lactobacillus delbrueckii* und allen seinen Unterarten, *Lactobacillus gasseri, Lactobacillus johnsonii, Streptococcus thermophilus, Lactobacillus delbrueckii subsp. bulgaricus, gegebenenfalls assoziiert mit Streptococcus thermophilus; Lactobacillus fermentum, Lactobacillus brevis, Lactobacillus lactis subsp. lactis, Lactococcus lactis* subsp. *cremoris und Leuconostoc-Arten; Enterococcus faecium, Pediococcus pentosaceus, Pediococcus acidilactici; Bi*fidobakterien, wie *Bifidobacterium longum, Bifidobacterium breve, Bifidobacterium bifidum, Bifidobacterium infantis, Bifidobacterium lactis; und*/*oder Propionibakterium-Arten, Hefe*arten und/oder Schimmelpilzarten; wobei die obenstehenden Arten lebend und lebensfähig und/oder abgetötet sind und wobei die Arten in mikrobiellen Kulturen in einer getrockneten konzentrierten Form mit einem Konzentrationsbereich, der von 10⁶ KbE/g bis 10¹¹ KbE/g reicht, vorliegen,
wobei die Zusammensetzungen auch **dadurch gekennzeichnet sind, dass** sie verschiedene Stämme der gleichen Art mit einer unterschiedlichen Empfindlichkeit gegenüber Bakteriophagen und mit den gleichen biologischen und probiotischen Eigenschaften umfassen.

2. Zusammensetzungen nach Anspruch 1, **dadurch gekennzeichnet, dass** sie auch wenigstens eine der folgenden Komponenten umfassen: andere Mikroorganismen, Enzyme, Mineralsalze, Vitamine, Präbiotika, natürliche Fasern bzw. Ballaststoffe, Phytoderivate, Antioxidantien, fermentierte Milch, Breie, Nahrungs- bzw. Futtermittel.

3. Zusammensetzungen nach Anspruch 1, **dadurch gekennzeichnet, dass** sie Präbiotika, wie natürliche Fasern bzw. Ballaststoffe umfassen.

4. Zusammensetzungen nach Anspruch 1, **dadurch gekennzeichnet, dass** wenigstens einer der probiotischen Mikroorganismen in einer Konzentration von weniger als 10⁹ KbE/g vorliegt.

5. Zusammensetzungen nach Anspruch 1, **dadurch gekennzeichnet, dass** sie *Streptococcus thermophilus*, Bifidobakterien, wie *Bifidobacterium longum, Bifidobacterium breve, Bifidobacterium infantis, Bifidobacterium lactis, Bifidobacterium bifidum, Lactobacillus acidophilus* in einer Konzentration, die von 10⁹ bis 10¹¹ KbE/g reicht, *Lactobacillus plantarum, Lactobacillus casei* subsp. *casei, Lactobacillus delbrue*ckii subsp. *bulgaricus, Enterococcus faecium* in einer Konzentration, die von 10⁶ bis 10⁹ KbE/g reicht, umfassen.

6. Zusammensetzungen nach Anspruch 1, **dadurch gekennzeichnet, dass** die lebenden und lebensfähigen und/oder abgetöteten Hefen Hefen mit einer geringen Fermentationsfähigkeit zur probiotischen Verwendung sind, reich an essentiellen Aminosäuren.

7. Zusammensetzungen nach Anspruch 6, **dadurch gekennzeichnet, dass** die Hefe *Saccharomyces cerevisiae* oder *Saccharomyces boulardii* ist.

8. Zusammensetzungen nach Anspruch 2, **dadurch gekennzeichnet, dass** die natürlichen Enzyme aus einem Gemisch, hergestellt aus β-Glucanase und Xylanase, produziert durch Mikroorganismen des Trichoderma-Typs, bestehen.

9. Zusammensetzungen nach Anspruch 2, **dadurch gekennzeichnet, dass** die natürlichen Fasern bzw. Ballaststoffe ausgewählt sind aus Fasern bzw. Ballaststoffen von Gummi arabicum, Hafer, Äpfeln, Inulin, Psyllium, mikrokristalliner Cellulose.

10. Zusammensetzungen nach Anspruch 2, **dadurch gekennzeichnet, dass** die Antioxidantien natürliche Antioxidantien sind.

11. Zusammensetzungen nach Anspruch 10, **dadurch gekennzeichnet, dass** die natürlichen Antioxidantien ausgewählt sind aus Oleuropein (aus extravirginem Olivenöl), Lycopin (aus Tomaten), Bioflavonoiden (aus Zitrusfrüchten), Phenolkomponenten (aus roten Trauben).

12. Zusammensetzungen nach Anspruch 2, **dadurch gekennzeichnet, dass** die Vitamine und Mineralsalze ausgewählt sind aus Vitamin A, B1, B2, B6, B12, Niacin, C, D3, E, Folsäure, Kalzium, Phosphor, Magnesium, Eisen, Zink.

13. Zusammensetzungen nach Anspruch 2, **dadurch gekennzeichnet, dass** die Phytoderivate ausgewählt sind aus jenen, die aus Eleuterococcus und grünem Tee extrahiert werden.

14. Zusammensetzungen nach Anspruch 1, **gekennzeichnet durch** das Vorhandensein von *S. thermophilus* und *L. delbrueckii* subsp. *bulgaricus,* entwickelt in symbiotischer Assoziation (oder Protokooperation).

15. Verwendung der Zusammensetzungen nach einem der Ansprüche 1 bis 14 zur Herstellung von Integratoren, Nahrungsmitteln und/oder diätetisch-therapeutischen Produkten, Getränken und/oder Nahrungs- bzw. Futtermittel für die menschliche und/oder Tierernährung.

16. Verwendung nach Anspruch 15, wobei die Nahrungsprodukte Milch, Käse, Brei, homogenisierte Produkte (basierend auf Fleisch, Milch, Käse, Früchten, Gemüsen), diätetische Nahrungsmittelprodukte, bestimmt für Diabetiker, wie zum Beispiel Marmeladen, Schokolade, Süßungsmittel, außer Saccharose, oder Tierfutter sind.

17. Verwendung nach Anspruch 15, wobei die Milch eine fermentierte oder nicht-fermentierte Milch ist, mit dem direkten Inoculum probiotischer Mikroorganismen in einer getrockneten konzentrierten Form.

18. Verwendung nach Anspruch 15, wobei der Käse ein therapeutischer diätetischer Käse ist, erhalten durch die Zugabe von probiotischen Mikroorganismen in einer getrockneten konzentrierten Form in einer bestimmten Bearbeitungsstufe des Käses.

19. Verwendung nach Anspruch 15, wobei die Getränke Instantgetränke oder Wasser, enthaltend die Zusammensetzungen nach einem der Ansprüche 1 bis 14, sein können.

20. Integratoren, Nahrungsmittel und/oder diätetischtherapeutische Produkte, Getränke und/oder Nahrungs- bzw. Futtermittel zur menschlichen und/oder Tierernährung, **dadurch gekennzeichnet, dass** sie eine Zusammensetzung nach einem der Ansprüche 1 bis 14 enthalten.

## Revendications

1. Compositions diététiques et/ou pharmaceutiques pour une utilisation chez l'homme et/ou les animaux, à base de cultures microbiennes comprenant des espèces autochtones et allochtones par rapport à l'homme et aux animaux, choisies parmi les espèces de bactéries de l'acide lactique *Lactobacillus acidophilus, Lactobacillus plantarum, Lactobacillus casei* subsp. *casei, Lactobacillus casei* subsp. *rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii* subsp. *delbrueckii, Lactobacillus delbrueckii* et toutes leurs sous-espèces, *Lactobacillus gasseri, Lactobacillus johnsonii, Streptococcus thermophilus, Lactobacillus delbrueckii* subsp. *bulgaricus,* le cas échéant associées à *Streptococcus thermophilus ; Lactobacillus fermentum, Lactobacillus brevis, Lactococcus lactis* subsp. *lactis, Lactococcus lactis* subsp. *cremoris* et *Leuconostoc* sp. ; *Enterococcus faecium, Pediococcus pentosaceus, Pediococcus acidilactici ; Bifidobaceries* comme par exemple *Bifidobacterium longum, Bifidobacterium breve, Bifidobacterium bifidum, Bifidobacterium infantis, Bifidobacterium lactis ;* et/ou des espèces de propionibactéries, des espèces de levures et/ou des espèces de moisissures ; les espèces précitées étant vivantes et viables et/ou dévitalisées et lesdites espèces étant présentes dans les cultures microbiennes sous une forme concentrée séchée à une concentration allant de 10⁶ ufc/g à 10¹¹ ufc/g,
lesdites compositions étant également **caractérisées en ce qu'**elles comprennent différentes souches de la même espèce ayant une sensibilité différente aux bactériophages et ayant les mêmes propriétés biologiques et probiotiques.

2. Compositions selon la revendication 1, **caractérisées en ce qu'**elles comprennent également un au moins des composants suivants : d'autres micro-organismes, des enzymes, des sels minéraux, des vitamines, des prébiotiques, des fibres naturelles, des phytodérivés, des antioxydants, du lait fermenté, des bouillies, des aliments pour animaux.

3. Compositions selon la revendication 1, **caractérisées en ce qu'**elles comprennent des prébiotiques tels que des fibres naturelles.

4. Compositions selon la revendication 1, **caractérisées en ce que** l'un au moins des micro-organismes probiotiques est présent à une concentration inférieure à 10⁹ ufc/g.

5. Compositions selon la revendication 1, **caractérisées en ce qu'**elles comprennent *Streptococcus thermophilus,* des bifidobactéries telles que *Bifidobacterium longum, Bifidobacterium breve, Bifidobacterium infantis, Bifidobacterium lactis, Bifidobacterium bifidum, Lactobacillus acidophilus* à une concentration allant de 10⁹ à 10¹¹ ufc/g, *Lactobacillus plantarum, Lactobacillus casei* subsp. *casei, Lactobacillus delbrueckii* subsp. *Bulgaricus, Enterococcus faecium* à une concentration allant de 10⁶ à 10⁹ ufc/g.

6. Compositions selon la revendication 1, **caractérisées en ce que** les levures vivantes et viables et/ou dévitalisées sont des levures ayant une faible capacité de fermentation pour un usage probiotique et riches en acides aminés essentiels.

7. Compositions selon la revendication 6, **caractérisées en ce que** la levure est *Saccharomyces cerevisiae* ou *Saccharomyces boulardii.*

8. Compositions selon la revendication 2, **caractérisées en ce que** les enzymes naturelles sont constituées d'un mélange composé de β-glucanase et de xylanase produites par des micro-organismes du type *Trichoderma.*

9. Compositions selon la revendication 2, **caractérisées en ce que** les fibres naturelles sont choisies parmi des fibres d'acacia, d'avoines, de pommes, d'inuline, de psyllium, de cellulose microcristalline.

10. Compositions selon la revendication 2, **caractérisées en ce que** les antioxydants sont des antioxydants naturels.

11. Compositions selon la revendication 10, **caractérisées en ce que** les antioxydants naturels sont choisis parmi l'oleuropéine (extraite de l'huile d'olive extra vierge), le lycopène (extrait de la tomate), des bioflavonoïdes (extraits d'agrumes), des composants phénoliques (extraits du raisin rouge).

12. Compositions selon la revendication 2, **caractérisées en ce que** les vitamines et les sels minéraux sont choisis parmi les vitamines A, B1, B2, B6, B12, la niacine, les vitamines C, D3, E, l'acide folique, le calcium, le phosphore, le magnésium, le fer, le zinc.

13. Compositions selon la revendication 2, **caractérisées en ce que** les phytodérivés sont choisis parmi ceux extraits *d'Eleuterococcus* et du thé vert.

14. Compositions selon la revendication 1, **caractérisées par** la présence de *S*. *thermophilus* et de *L. delbrueckii* subsp. *bulgaricus* développés dans une association symbiotique (ou protocoopération).

15. Utilisation des compositions selon l'une quelconque des revendications 1 à 14, pour préparer des intégrateurs, des denrées alimentaires et/ou des produits, boissons et/ou aliments diétético-thérapeutiques pour la nutrition humaine et/ou animale.

16. Utilisation selon la revendication 15, dans laquelle les produits alimentaires sont le lait, le fromage, des bouillies, des produits homogénéisés (à base de viande, de lait, de fromage, de fruit, de légumes), des produits alimentaires diététiques destinés aux diabétiques tels que des confitures, le chocolat, des édulcorants autres que le saccharose ou des aliments pour animaux.

17. Utilisation selon la revendication 15, dans laquelle le lait est un lait fermenté ou non fermenté, avec inoculation directe de micro-organismes probiotiques sous une forme concentrée séchée.

18. Utilisation selon la revendication 15, dans laquelle le fromage est un fromage diétético-thérapeutique obtenu en ajoutant des micro-organismes probiotiques sous une forme concentrée séchée dans une phase d'élaboration donnée du fromage.

19. Utilisation selon la revendication 15, dans laquelle les boissons peuvent être des boissons instantanées ou de l'eau contenant les compositions selon l'une quelconque des revendications 1 à 14.

20. Intégrateurs, denrées alimentaires et/ou produits, boissons et/ou aliments diétético-thérapeutiques pour la nutrition humaine et/ou animale, **caractérisés en ce qu'**ils contiennent une composition selon l'une quelconque des revendications 1 à 14.
